# NOUVEAU FASCICULE DE BREVET EUROPEEN

(11) **EP 0 756 865 B2**
(45) Date de publication et mention de la décision concernant l'opposition: **02.01.2002**
(45) Mention de la délivrance du brevet: 24.06.1998
(21) Numéro de dépôt: 96401524.2
(22) Date de dépôt: 10.07.1996
(51) Int. Cl.: A61K 7/48, A61K 7/02, A61K 7/027, A61K 7/025

(54) **Composition sous forme de pâte souple et procédé de préparation**
Zubereitung in Form von einer weichen Paste und Herstellungsverfahren
Composition in form of a supple paste and preparation process

(30) Priorité: 28.07.1995 FR 9509253
(43) Date de publication de la demande: 05.02.1997
(73) Titulaire: L'OREAL, 75008 Paris (FR)
(72) Inventeur: Agostini, Isabelle, 92290 Chatenay Malabry (FR); Miguel-Colombel, Dolorès, 94240 L Hay les Roses (FR); Pradier, François, 92260 Fontenay aux Roses (FR); Junino, Alex, 93190 Livry Gargan (FR); Lebras-Roulier, Véronique, 75002 Paris (FR)
(74) Mandataire: Dodin, Catherine

(56) Documents cités:
- EP-A- 0 133 963
- EP-A- 0 133 964
- EP-A- 0 197 485
- EP-A- 0 268 950
- EP-A- 0 310 252
- EP-A- 0 549 267
- EP-A- 0 602 905
- FR-A- 2 646 346
- FR-A- 2 707 485
- JP-A- 6 165 809
- US-A- 4 699 780
- US-A- 4 822 603
- US-A- 4 874 868
- US-A- 4 919 934
- US-A- 4 990 561
- US-A- 5 093 107
- US-A- 5 254 542
- US-A- 5 288 482
- US-A- 5 382 432
- PATENT ABSTRACTS OF JAPAN vol. 012, no. 198 (C-502), 8 juin 1988 & JP 62 298519 A (SHISEIDO CO LTD;OTHERS: 01), 25 décembre 1987,
- PATENT ABSTRACTS OF JAPAN vol. 010, no. 230 (C-365), 9 août 1986 & JP 61 065808 A (SHISEIDO CO LTD), 4 avril 1986,
- "Product information for Dow Corning 200 Fluid" 1990
- Karl Rothemann, Das Grosse Rezeptbuch, 1949, p. 499-503
- M.S. Balsam et al, Cosmetics, Science and Technology, 2nd ed., vol. 1, 1972, Wiley-Interscience
- E.J. Better et al, Taschenbuch für die Wachs-Industrie, 1932, Wissenschaftliche Verlagsgesellschaft m.b.H., Stuttgart, p. 186-189
- Lüdecke, C., Taschenbuch für die Wachsindutrie, 1958, Wissenchaftliche Verlagsgesellschaft m.b.H., Stuttgart, p. 476-477

## Description

La présente invention a trait à une composition notamment cosmétique se présentant sous forme d'une pâte souple, pouvant être utilisée pour le soin et/ou le maquillage des lèvres et/ou de la peau.

Les compositions cosmétiques pouvant être appliquées sur la peau ou les lèvres comme produit de maquillage ou de soin, telles que les bases pour lèvres ou les rouges à lèvres par exemple, contiennent généralement des corps gras dont des cires, des pigments et/ou charges et, éventuellement, des additifs. Il est connu que plus la quantité de cires présente dans la composition est importante, plus celle-ci a une consistance ferme, ce qui permet son utilisation sous forme de bâton. Or la présentation, en particulier d'un rouge à lèvres, sous forme de bâton présente certains inconvénients: le dessin des contours des lèvres est malaisé et la tenue du bâton à la chaleur n'est pas optimale. Il est également connu des compositions cosmétiques se présentant sous forme de pâte souple, applicable à l'aide d'un pinceau, par exemple. Ces compositions ne contiennent généralement pas ou peu de cires, notamment en une faible quantité de l'ordre de 8-12%, ce qui permet de les prélever et de les appliquer aisément, étant donné qu'une quantité plus importante des cires conduirait à une composition de viscosité plus élevée qui serait alors inapplicable.
Toutefois, les cires permettent d'apporter certaines propriétés intéressantes aux compositions, en particulier des qualités d'onctuosité, et de tenue et d'épaisseur du film.
D'autre part, on a constaté que les compositions de l'art antérieur présentent l'inconvénient de transférer, c'est-à-dire de se déposer au moins en partie, en laissant une trace, sur certains supports avec lesquels elles peuvent être mises en contact, notamment un verre, un vêtement ou la peau. Il s'en suit une persistance médiocre du film sur les lèvres d'où la nécessité de renouveler régulièrement l'application de la composition de rouge à lèvres.

Il est connu des documents EP-A-602 905, EP-A-268 950 et JP-A-6165808 des compositions sans transfert ou de bonne tenue, contenant des cires et/ou des huiles phénylées associées à des silicones volatiles. Toutefois, ces compositions ne se présentent pas sous forme de pâte souple avec un haut taux de cires.

Ainsi il subsiste le besoin d'une composition se présentant sous la forme d'une pâte souple tout en contenant une quantité importante de cires par rapport à l'état de la technique, et qui présente des propriétés de transfert réduites.

La présente invention a pour but de pallier ce besoin en proposant une composition qui possède les caractéristiques souhaitées.

Un objet de la présente invention est donc une composition se présentant sous forme d'une pâte souple selon la revendication 1.
Un autre objet est un procédé de préparation de cette composition selon la revendication 16.
Un autre objet de l'invention est l'utilisation selon l'une des revendications 20 ou 21 afin de diminuer le transfert et/ou la migration de ladite composition et/ou afin d'améliorer la tenue de ladite composition.
Un autre objet de l'invention est un procédé pour limiter, diminuer et/ou empêcher le transfert d'une composition de maquillage ou de soin de la peau, des muqueuses et/ou des semi-muqueuses, notamment un rouge à lèvres ou un fond de teint, selon la revendication 31.

La composition selon la présente invention est donc une pâte souple, dont on peut mesurer la viscosité, par opposition à la structure solide d'un bâton, ou stick, dont on ne peut pas mesurer la viscosité. Ladite viscosité dynamique à 25°C est généralement comprise entre 3 et 35 Pa.s, mesurée avec un viscosimètre rotatif CONTRAVES TV équipé d'un mobile "MS-r4" à la fréquence de 60 Hz.
On a de plus constaté que le film obtenu avec la composition selon l'invention était de bonne tenue et ne présentait pas de problème de migration. Le film n'a en effet pas tendance à se propager à l'intérieur des ridules de la peau qui entourent les lèvres, en créant un effet inesthétique.
L'invention s'applique aux compositions à appliquer sur la peau, les semi-muqueuses et/ou les muqueuses, et notamment, non seulement aux produits de maquillage des lèvres mais aussi aux produits de soin des lèvres ainsi qu'aux produits de maquillage et de soin de la peau tels que les fonds de teint. On considère notamment comme muqueuse, la partie interne de la paupière inférieure; parmi les semi-muqueuses, on entend plus particulièrement les lèvres du visage.

La composition selon l'invention comprend donc une phase grasse dans laquelle sont présentes au moins une cire, au moins une huile volatile et au moins une huile siliconée phénylée.
La cire entrant dans la composition présente de préférence un point de fusion supérieur à 45°C environ, et en particulier supérieur à 55°C, et/ou un indice de pénétration de l'aiguille à 25°C de préférence compris entre 3 et 40. Parmi les cires envisageables, seules ou en mélange, on peut citer les cires animales, végétales, minérales et synthétiques, telles que la cire d'abeilles; la cire de Carnauba, de Candellila, d'Ourricoury, du Japon, les cires de fibres de lièges ou de canne à sucre; les cires de paraffine, de lignite; les cires microcristallines; les ozokérites; les cires de polyéthylène et les cires obtenues par synthèse de Fischer-Tropsch; les cires de silicone. La composition comprend de préférence 15-60% en poids de cires ayant un point de fusion supérieur à 55°C. Ainsi, la composition selon l'invention ayant une teneur en cires élevée, supérieur à 12% en poids, elle est susceptible de former un film de bonne tenue lorsqu'elle est appliquée en couche, notamment sur les lèvres.

La composition selon l'invention comprend également une huile volatile, qui peut être choisie en particulier parmi les huiles hydrocarbonées ou les huiles siliconées, cycliques ou linéaires, seules ou en mélange. Par huile volatile, on entend dans la présente description, toute huile susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C. Parmi les huiles siliconées volatiles, on peut citer la cyclotétradiméthylsiloxane, la cyclopentadiméthylsiloxane, la cyclohexadiméthylsiioxane et le méthylhexyldiméthylsiloxane. Parmi les huiles hydrocarbonées volatiles, on peut citer les isoparaffines.
La composition selon l'invention peut comprendre 8-70% en poids, de préférence 30-60%, d'huiles volatiles par rapport au poids total de la composition.

La composition selon l'invention comprend également au moins une huile siliconée phénylée. Cette huile peut être un polyphénylméthylsiloxane ou un phényltriméthicone, ou un mélange de différentes huiles siliconées phénylées, et en particulier peut répondre à la formule suivante : 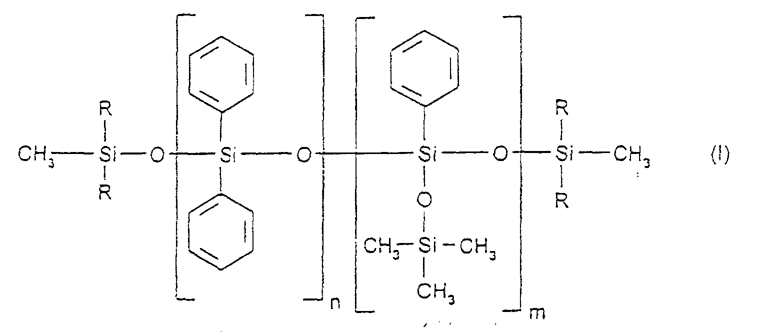 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.
De préférence, R est un radical méthyle, éthyle, propyle, isopropyle, décyle, dodécyle ou octadécyle, ou encore un radical phényle, tolyle, benzyle ou phénéthyle.

Parmi ces huiles phénylées, on peut citer l'huile Belsil PDM1000 de Wacker, les huiles DC556 ou SF558 de Dow Corning, l'huile Abil AV8853 de Goldschmidt ou l'huile Silbione 70633V30 de Rhône Poulenc.
La composition selon l'invention peut comprendre 1-35% en poids, de préférence 10-20%, d'huiles siliconées phénylées.

La composition peut en outre comprendre d'autres corps gras en plus de ceux cités ci-dessus, usuellement utilisés dans le domaine considéré, et notamment des huiles, des gommes et/ou des corps gras pâteux d'origine végétale, minérale, animale, synthétique ou siliconée.
Parmi les corps gras siliconés, on peut citer les polydiméthylsiloxanes (PDMS) et les alkyldiméthicones, ainsi que les silicones modifiées par des groupements aliphatiques et/ou aromatiques, éventuellement fluorés, ou par des groupements fonctionnels tels que des groupements hydroxyles, thiols et/ou amines.
Parmi les corps gras non siliconés, on peut citer l'huile de paraffine, de vaseline, le perhydrosqualène, l'huile d'arachide, d'amande douce, de calophyllum, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides; des huiles hydrogénées concrètes à 25°C; des lanolines; des esters gras concrets à 25°C.
Ces corps gras peuvent en particulier être choisis de manière variée par l'homme du métier afin de préparer une composition ayant les propriétés souhaitées, par exemple en consistance ou en texture.

Toutefois, de préférence, la composition selon l'invention comprend moins de 20% en poids d'huile hydrocarbonée non volatile, et par exemple moins de 5% en poids, voire pas du tout. En effet, on a constaté que dans ce cas, les propriétés de non transfert' de la composition obtenue étaient encore améliorées.

La composition peut comprendre également une phase particulaire, généralement présente à raison de 0-35% en poids, de préférence 5-25% en poids, et qui peut comprendre des pigments et/ou des nacres et/ou des charges habituellement utilisés dans les compositions cosmétiques.
Par pigments, il faut comprendre des particules blanches ou colorées, minérales ou organiques, insolubles dans la cire et la silicone volatile, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition et/ou de la douceur, de la matité et de l'uniformité au maquillage. Par nacres, il faut comprendre des particules irisées qui réfléchissent la lumière.
Les pigments peuvent être présents dans la composition à raison de 0-30% en poids de la composition finale, et de préférence à raison de 10-15%. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.
Les nacres peuvent être présentes dans la composition à raison de 0-20% en poids, de préférence à un taux élevé de l'ordre de 8-15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présemes à raison de 0-30% en poids, de préférence 5-15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le micatitane, le nitrure de bore, les microsphères telles que l'Expancel (Nobel Industrie), le polytrap (Dow Corning) et les microbilles de résine de silicone (Tospearls de Toshiba).

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des sphingolipides, des agents autobronzants tels que la DHA, des filtres solaires, des tensioactifs, des polymères liposolubles notamment hydrocarbonés, tels que le polybutène, les polyalkylènes, les polyacrylates et les polymères siliconés compatibles avec les corps gras.
Bien entendu l'homme du métier veillera à choisir les éventuels composés complémentaires, et/ou leur quantité, de telle manière que les propriétés avantageuses de la composition selon l'invention ne soient pas, ou substantiellement pas, altérées par l'adjonction envisagée.

La composition selon l'invention est préparée de la manière suivante.
On prépare tout d'abord un prémélange comprenant au moins une partie des différents constituants de la composition, dont au moins la ou les cires, on chauffe ce prémélange à une température à laquelle il fond, on ajoute le cas échéant le reste des constituants tout en malaxant le mélange obtenu, puis l'on refroidit le mélange obtenu tout en le malaxant pendant son refroidissement, le malaxage étant effectué au moins en partie dans un mélangeur-extrudeur. Le refroidissement est effectué jusqu'à température ambiante, température à laquelle on peut ajouter l'huile volatile. Toutefois, ladite huile volatile peut également être ajoutée pendant l'étape de refroidissement, de préférence à une température inférieure ou égale à 45°C environ.
Ce procédé permet d'obtenir une composition se présentant sous forme de pâte souple et homogène, bien qu'elle contienne des cires et éventuellement une phase particulaire, en une quantité importante.
De plus, cette pâte est de qualité constante et reproductible.

On peut effectuer l'opération de chauffage selon toute technique connue.
Dans un mode particulier de réalisation de l'invention, les opérations de chauffage et de malaxage, voire de refroidissement, sont réalisées en totalité dans un ou plusieurs extrudeurs, mono ou bivis, disposés à la suite les uns des autres, et de préférence dans un extrudeur bivis unique.
De plus, il est possible, en adaptant la filière de sortie du mélangeur-extrudeur, de conditionner la composition en ligne à la sortie dudit mélangeur-extrudeur.

On a en effet constaté que la composition obtenue après extrusion présente une douceur particulière, et procure une certaine sensation de glissant lorsqu'elle est appliquée sur la peau, tout en évitant l'aspect et la sensation de gras huileux.
Les conditions dans lesquelles l'extrusion peut être effectuée sont décrites dans la demande de brevet FR94-00756.

Les compositions selon l'invention peuvent se présenter sous la forme d'une composition cosmétique et notamment sous forme d'un produit de maquillage de la peau, en particulier un fond de teint, un fard à joues ou à paupières, ou un rouge à lèvres. Elles peuvent également se présenter sous forme non colorée, contenant éventuellement des actifs cosmétiques. Elles peuvent alors notamment être utilisées comme base de soin pour les lèvres, ou en tant que base fixante à appliquer sur un rouge à lèvres classique. La base fixante forme alors un film protecteur sur le film de rouge, en limite le transfert et la migration, et permet d'augmenter ainsi sa tenue. Les compositions peuvent encore se présenter sous la forme d'une composition dermatologique ou de soin de la peau, ou encore sous forme d'une composition solaire ou autobronzante.

L'invention est illustrée plus en détail dans les exemples suivants.

### Exemple 1

On prépare un rouge à lèvres sous forme de pâte souple, ayant la composition suivante:

| | |
|---|---|
| . cyclopentadiméthylsiloxane | 40 g |
| . polyphénylméthylsiloxane (DC556 Fluid de Dow Corning) | 15 g |
| . corps gras siliconé pâteux | 5 g |
| . cires (silicone et polyéthylène) | 25 g |
| . pigments | 10 g |
| . charges | 5 g |

On introduit ces différents ingrédients, à l'exception de l'huile volatile, dans un extrudeur bi-vis, à une température d'environ 75-95°C en entrée.
On introduit l'huile volatile dans l'extrudeur en fin d'extrusion, à une température de l'ordre de 20-25°C.

On obtient en sortie une pâte souple de viscosité égale à 27 Pa.s, se présentant sous forme d'une seule phase staple et homogène, et pouvant être prélevée à l'aide d'un pinceau pour son application.

Cette composition permet l'obtention d'un film homogène, facilement applicable et s'étalant facilement et uniformément. Le film obtenu présente également une texture légère et reste confortable à porter tout au long de la journée.

On applique cette composition sur la partie gauche des lèvres de plusieurs personnes.
Pour comparaison, on applique sur la partie droite desdites lèvres, un rouge à lèvres sous forme de pâte souple classique, ne comprenant ni huile de silicone volatile, ni huile de silicone phénylée.
On laisse sécher les rouges à lèvres à température ambiante pendant 5 minutes, puis on applique la totalité des lèvres sur une feuille de papier.
On constate sur la totalité des feuilles de papier, une trace importante de rouge laissée par la composition selon l'art antérieur.
La composition selon l'invention laisse sur la feuille une trace très faible, à peine perceptible.

### Exemple 2

On prépare une base fixante pour rouge à lèvres sous forme de pâte souple, ayant la composition suivante:

| | |
|---|---|
| . cyclopentadiméthylsiloxane | 40 g |
| . polyphénylméthylsiloxane (DC556 Fluid de Dow Corning) | 15 g |
| . corps gras pâteux siliconé | 5 g |
| . cires (silicone et polyéthylène) | 25 g |
| . charges | 15 g |

La préparation s'effectue selon l'exemple 1.

Le film obtenu par application de la composition est homogène et facilement applicable, en s'étalant facilement et uniformément sur un rouge à lèvres classique.

## Revendications

1. Composition se présentant sous forme d'une pâte souple et comprenant, dans une phase grasse, une huile volatile, une huile siliconée phénylée et au moins 12% en poids de cire par rapport au poids total de la composition, la composition étant obtenue par prémélange d'au moins une partie des constituants, dont au moins la ou les cires, chauffage de ce prémélange à une température à laquelle il fond, malaxage du mélange obtenu pendant son refroidissement jusqu'à température ambiante, le malaxage étant effectué au moins en partie dans un mélangeur-extrudeur.

2. Composition selon la revendication 1, dans laquelle l'huile volatile est choisie parmi les huiles hydrocarbonées ou les huiles siliconées, cycliques ou linéaires, seules ou en mélange.

3. Composition selon l'une des revendications précédentes, dans laquelle l'huile volatile est choisie parmi la cyclotétradiméthylsiloxane, la cyclopentadiméthylsiloxane, la cyclohexadiméthylsiloxane, le méthylhexyldiméthylsiloxane et les isoparaffines, seules ou en mélange.

4. Composition selon l'une des revendications précédentes, dans laquelle l'huile volatile est présente à raison de 8-70% en poids, de préférence 30-60%, par rapport au poids total de la composition.

5. Composition selon l'une des revendications précédentes, dans laquelle l'huile phénylée siliconée est choisie parmi les huiles de formule (I) et leur mélange: 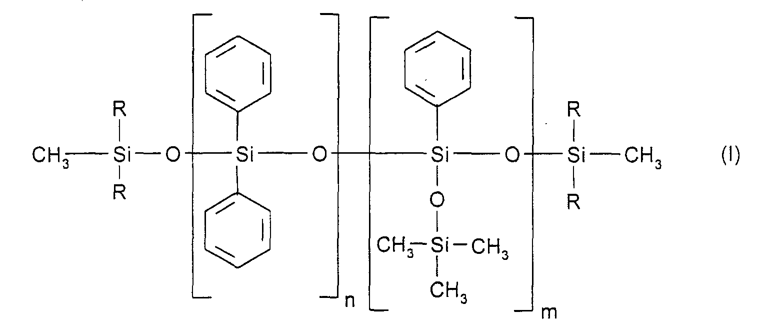 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.

6. Composition selon la revendication 5, dans laquelle l'huile siliconée phénylée est choisie parmi les huiles de formule (I) pour lesquelles R est un radical méthyle, éthyle, propyle, isopropyle, décyle, dodécyle, octadécyle, phényle, tolyle, benzyle ou phénéthyle.

7. Composition selon l'une des revendications précédentes, dans laquelle l'huile siliconée phénylée est présente à raison de 1-35% en poids, de préférence 10-20%, dans la composition.

8. Composition selon l'une des revendications précédentes, dans laquelle la cire présente un point de fusion supérieur à 45°C environ, de préférence supérieur à 55°C et/ou un indice de pénétration de l'aiguille, à 25°C, compris entre 3 et 40.

9. Composition selon l'une des revendications précédentes, dans laquelle la cire est choisie parmi les cires animales, végétales, minérales et synthétiques, telles que la cire d'abeilles; la cire de Carnauba, de Candellila, d'Ourricoury, du Japon, les cires de fibres de lièges ou de canne à sucre; les cires de paraffine, de lignite; les cires microcristallines; les ozokérites; les cires de polyéthylène, les cires de polyéthylène; les cires obtenues par synthèse de Fischer-Tropsch; les cires de silicone; et leurs mélanges.

10. Composition selon l'une des revendications précédentes, dans laquelle la cire est présente à raison de 15-60% en poids par rapport au poids total de la composition.

11. Composition selon l'une des revendications précédentes, présentant une viscosité dynamique à 25°C comprise entre 3 et 35 Pa.s, mesurée avec un viscosimètre rotatif CONTRAVES TV équipé d'un mobile "MS-r4" à la fréquence de 60 Hz.

12. Composition selon l'une des revendications précédentes, comprenant moins de 20% en poids, de préférence moins de 5% en poids, d'huile hydrocarbonée non volatile.

13. Composition selon l'une des revendications précédentes, comprenant en outre une phase particulaire présente à raison de 0-35% en poids, de préférence 5-25% en poids.

14. Composition selon l'une des revendications précédentes se présentant sous la forme d'une composition cosmétique ou dermatologique, comprenant un support cosmétiquement ou dermatologiquement acceptable.

15. Composition selon l'une des revendications précédentes se présentant sous la forme d'une composition de maquillage, notamment un fond de teint, un fard à joues ou à paupières, ou un rouge à lèvres; d'une base de soin ou d'une base fixante pour les lèvres; d'un produit de soin de la peau ou d'une composition solaire ou autobronzante.

16. Procédé de préparation d'une composition selon l'une quelconques des revendications précédentes, dans lequel on prépare un prémélange comprenant au moins une partie des différents constituants de la composition, dont au moins la ou les cires, on chauffe ce prémélange à une température à laquelle il fond, on ajoute le cas échéant le reste des constituants tout en malaxant le mélange obtenu, puis l'on refroidit le mélange obtenu tout en le malaxant pendant son refroidissement jusqu'à température ambiante, le malaxage étant effectué au moins en partie dans un mélangeur-extrudeur.

17. Procédé selon la revendication 16, dans lequel les huiles volatiles sont ajoutées pendant l'étape de refroidissement, de préférence à une température inférieure ou égale à 45°C environ.

18. Procédé selon l'une des revendications 16 à 17, dans lequel les opérations de chauffage, de malaxage et de refroidissement sont réalisées dans un ou plusieurs extrudeurs disposés à la suite les uns des autres.

19. Procédé selon l'une des revendications 16 à 18, dans lequel les opérations de chauffage, de malaxage et de refroidissement sont réalisées dans un extrudeur bivis unique.

20. Utilisation de l'association d'une huile volatile et d'une huile siliconée phénylée dans une composition sous forme de pâte souple, comprenant au moins 12% en poids de cire par rapport au poids total de la composition, afin de diminuer le transfert et/ou la migration de ladite composition.

21. Utilisation de l'association d'une huile volatile et d'une huile siliconée phénylée dans une composition sous forme de pâte souple, comprenant une phase grasse contenant au moins 12 % de cire par rapport au poids total de la composition, afin d'améliorer la tenue de ladite composition.

22. Utilisation selon l'une des revendications 20 à 21, dans laquelle l'huile volatile est choisie parmi les huiles hydrocarbonées ou les huiles siliconées, cycliques ou linéaires, seules ou en mélange.

23. Utilisation selon l'une des revendications 20 à 22, dans laquelle l'huile volatile est présente à raison de 8-70% en poids, de préférence 30-60%, dans la composition.

24. Utilisation selon l'une des revendications 20 à 23, dans laquelle l'huile siliconée phénylée est choisie parmi les huiles de formule (I) et leurs mélanges, : 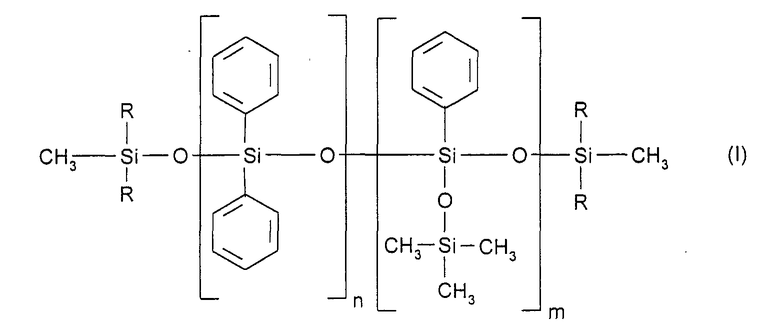 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.

25. Utilisation selon l'une des revendications 20 à 24, dans laquelle l'huile siliconée phénylée est présente à raison de 1-35% en poids, de préférence 10-20%, dans la composition.

26. Utilisation selon l'une des revendications 20 à 25, dans laquelle la cire est choisie parmi les cires animales, végétales, minérales et synthétiques, telles que la cire d'abeilles; la cire de Carnauba, de Candellila, d'Ourricoury, du Japon, les cires de fibres de lièges ou de canne à sucre; les cires de paraffine, de lignite; les cires microcristallines; les ozokérites; les cires de polyéthylène; les cires obtenues par synthèse de Fischer-Tropsch; les cires de silicone; et leurs mélanges.

27. Utilisation selon l'une des revendications 20 à 26, dans laquelle la cire est présente à raison de 15-60% en poids par rapport au poids total de la composition.

28. Utilisation selon l'une des revendications 20 à 27, dans une composition comprenant moins de 20% en poids, de préférence moins de 5% en poids, d'huile hydrocarbonée non volatile.

29. Utilisation selon l'une des revendications 20 à 28, dans une composition se présentant sous la forme d'une composition cosmétique ou dermatologique, comprenant un support cosmétiquement ou dermatologiquement acceptable.

30. Utilisation selon l'une des revendications 20 à 29 dans une composition se présentant sous la forme d'une composition de maquillage, notamment un fond de teint, un fard à joues ou à paupières, ou un rouge à lèvres; d'une base de soin ou d'une base fixante pour les lèvres; d'un produit de soin de la peau ou d'une composition solaire ou autobronzante.

31. Procédé pour limiter, diminuer et/ou empêcher le transfert d'une composition de maquillage ou de soin de la peau, des muqueuses et/ou des semi-muqueuses, notamment un rouge à lèvres ou un fond de teint, ladite composition se présentant sous forme d'une pâte souple et comprenant au moins 12% en poids de cire par rapport au poids total de la composition, consistant à introduire dans ladite composition une huile volatile et une huile siliconée phénylée.

## Patentansprüche

1. Zusammensetzung, die in Form einer weichen Paste vorliegt und die in einer Fettphase ein flüchtiges Öl, ein Phenylgruppenhaltiges Siliconöl und mindestens 12 Gew.-% Wachs, bezogen auf das Gesamtgewicht der Zusammensetzung, enthält, wobei die Zusammensetzung durch Vormischung mindestens eines Teils der Bestandteile, darunter mindestens das oder die Wachse, Erwärmen dieses Vorgemischs auf eine Temperatur, bei der es schmilzt, Kneten der erhaltenen Mischung während ihrer Abkühlung bis auf Umgebungstemperatur erhalten wird, wobei das Kneten zumindest teilweise in einem Extrudermischer durchgeführt wird.

2. Zusammensetzung nach Anspruch 1, wobei das flüchtige Öl unter cyclischen oder geradkettigen Kohlenwasserstoffölen und cyclischen oder geradkettigen Siliconölen, einzeln oder in Form eines Gemischs, ausgewählt ist.

3. Zusammensetzung nach einem der vorhergehenden Ansprüche, wobei das flüchtige Öl unter Cyclotetradimethylsiloxan, Cyclopentadimethylsiloxan, Cyclohexadimethylsiloxan, Methylhexyldimethylsiloxan und Isoparaffinen, einzeln oder in Form eines Gemischs, ausgewählt ist.

4. Zusammensetzung nach einem der vorhergehenden Ansprüche, wobei das flüchtige Öl in einem Anteil von 8 bis 70 Gew.-%, vorzugsweise 30 bis 60 Gew.-%, bezogen auf das Gesamtgewicht der Zusammensetzung, enthalten ist.

5. Zusammensetzung nach einem der vorhergehenden Ansprüche, wobei das Phenylgruppen-haltige Siliconöl unter den Ölen der Formel (I) 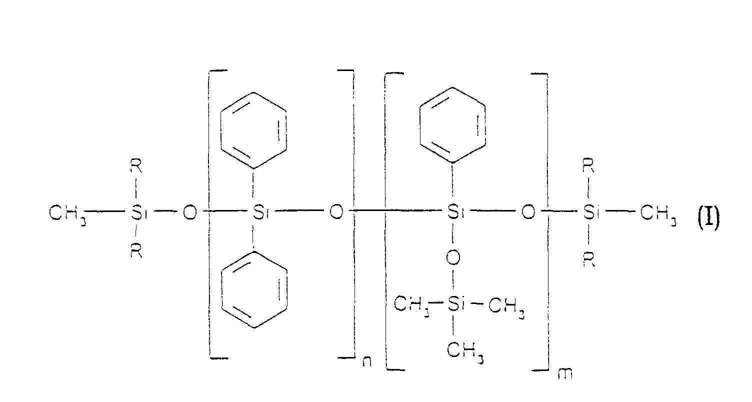 und deren Gemischen ausgewählt ist, in der bedeuten:
- R eine C₁₋₃₀-Alkylgruppe, eine Arylgruppe oder eine Aralkylgruppe,
- n eine ganze Zahl von 0 bis 100,
- m eine ganze Zahl von 0 bis 100 mit der Maßgabe, daß die Summe m+n 1 bis 100 beträgt.

6. Zusammensetzung nach Anspruch 5, wobei das Phenylgruppen-haltige Siliconöl unter den Ölen der Formel (I) ausgewählt ist, bei denen R Methyl, Ethyl, Propyl, Isopropyl, Decyl, Dodecyl, Octadecyl, Phenyl, Tolyl, Benzyl oder Phenethyl ist.

7. Zusammensetzung nach einem der vorhergehenden Ansprüche, wobei das Phenylgruppen-haltige Siliconöl in einem Anteil von 1 bis 35 Gew.-%, vorzugsweise 10 bis 20 Gew.-%, in der Zusammensetzung enthalten ist.

8. Zusammensetzung nach einem der vorhergehenden Ansprüche, wobei das Wachs einen Schmelzpunkt höher als etwa 45 °C, vorzugsweise höher als etwa 55 °C, und/oder eine Nadel-Penetrationszahl bei 25 °C von 3 bis 40 aufweist.

9. Zusammensetzung nach einem der vorhergehenden Ansprüche, wobei das Wachs unter tierischen, pflanzlichen, mineralischen und synthetischen Wachsen, wie z.B. Bienenwachs, Carnaubawachs, Candelillawachs, Ouricourywachs, Japanwachs, Korkfaserwachs oder Zuckerrohrwachs, Paraffinwachsen, Lignit, mikrokristallinen Wachsen, Ozokeriten, Polyethylenwachsen, den durch Fischer-Tropsch-Synthese erzeugten Wachsen, Siliconwachsen und deren Gemischen, ausgewählt ist.

10. Zusammensetzung nach einem der vorhergehenden Ansprüchen, wobei das Wachs in einem Anteil von 15 bis 60 Gew.-%, bezogen auf das Gesamtgewicht der Zusammensetzung, enthalten ist.

11. Zusammensetzung nach einem der vorhergehenden Ansprüche, die eine dynamische Viskosität bei 25 °C von 3 bis 35 Pa·s aufweist, gemessen mit einem Rotationsviskosimeter CONTRAVES TV, das mit einem Drehkörper "MS-r4" ausgerüstet ist, bei der Frequenz 60 Hz.

12. Zusammensetzung nach einem der vorhergehenden Ansprüche, die weniger als 20 Gew.-%, vorzugsweise weniger als 5 Gew.-% nichtflüchtiges Kohlenwasserstofföl enthält.

13. Zusammensetzung nach einem der vorhergehenden Ansprüche, die außerdem eine aus Partikeln bestehende Phase enthält, die in einem Anteil von 0 bis 35 Gew.-%, vorzugsweise 5 bis 25 Gew.-%, enthalten ist.

14. Zusammensetzung nach einem der vorhergehenden Ansprüche, die in Form einer kosmetischen oder dermatologischen Zusammensetzung vorliegt, die einen kosmetisch oder dermatologisch akzeptablen Träger enthält.

15. Zusammensetzung nach einem der vorhergehenden Ansprüche, die in Form einer Schminkzusammensetzung, insbesondere als Make-up, Wangen-Rouge oder Lidschatten oder ein Lippenstift, einer Pflegegrundmasse oder einer fixierenden Grundmasse für die Lippen, eines Pflegeprodukts für die Haut, einer Sonnenschutzzusammensetzung oder einer selbstbräunenden Zusammensetzung vorliegt.

16. Verfahren zur Herstellung einer Zusammensetzung nach einem der vorhergehenden Ansprüche, das folgende Schritte umfaßt: Herstellen eines Vorgemischs, das mindestens einen Teil der verschiedenen Bestandteile der Zusammensetzung, darunter mindestens das oder die Wachse, enthält, Erwärmen dieses Vorgemischs auf eine Temperatur, bei der es schmilzt, gegebenenfalls Hinzugeben des Rests der Bestandteile, währenddessen das erhaltene Gemisch geknetet wird, anschließend Abkühlen des erhaltenen Gemischs, wobei während seiner Abkühlung das Kneten fortgesetzt wird, wobei das Kneten mindestens teilweise in einem Extrudermischer durchgeführt wird.

17. Verfahren nach Anspruch 16, wobei die flüchtigen Öle während des Abkühlschritts, vorzugsweise bei einer Temperatur unterhalb oder gleich etwa 45 °C, zugegeben werden.

18. Verfahren nach einem der Ansprüche 16 und 17, wobei das Erwärmen, das Kneten und das Abkühlen in einem oder mehreren hintereinander angeordneten Extrudern durchgeführt werden.

19. Verfahren nach einem der Ansprüche 16 bis 18, wobei das Erwärmen, das Kneten und das Abkühlen in einem einzigen Doppelschneckenextruder durchgeführt werden.

20. Verwendung der Kombination eines flüchtigen Öls und eines Phenylgruppen-haltigen Siliconöls in einer Zusammensetzung in Form einer weichen Paste, die mindestens 12 Gew.-% Wachs, bezogen auf das Gesamtgewicht der Zusammensetzung, enthält, zur Verringerung des Übergehens und/oder der Wanderung der Zusammensetzung.

21. Verwendung der Kombination eines flüchtigen Öls und eines Phenylgruppen-haltigen Siliconöls in einer Zusammensetzung in Form einer weichen Paste, die eine Fettphase aufweist, die mindestens 12 Gew.-% Wachs, bezogen auf das Gesamtgewicht der Zusammensetzung, enthält, zur Verbesserung der Haltbarkeit der Zusammensetzung.

22. Verwendung nach einem der Ansprüche 20 und 21, wobei das flüchtige Öl unter cyclischen oder geradkettigen Kohlenwasserstoffölen und cyclischen oder geradkettigen Siliconölen, einzeln oder in Form eines Gemischs, ausgewählt wird.

23. Verwendung nach einem der Ansprüche 20 bis 22, wobei das flüchtige Öl in einem Anteil von 8 bis 70 Gew.-%, vorzugsweise 30 bis 60 Gew.-%, in der Zusammensetzung enthalten ist.

24. Verwendung nach einem der Ansprüche 20 bis 23, wobei das Phenylgruppen-haltige Siliconöl unter den Ölen der Formel (I) 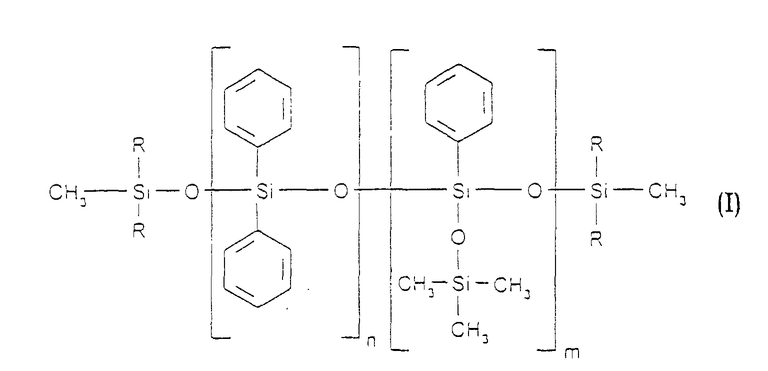 und deren Gemischen ausgewählt ist, in der bedeuten:
- R eine C₁₋₃₀-Alkylgruppe, eine Arylgruppe oder eine Aralkylgruppe,
- n eine ganze Zahl von 0 bis 100,
- m eine ganze Zahl von 0 bis 100 mit der Maßgabe, daß die Summe m+n 1 bis 100 beträgt.

25. Verwendung nach einem der Ansprüche 20 bis 24, wobei das Phenylgruppen-haltige Siliconöl in einem Anteil von 1 bis 35 Gew.-%, vorzugsweise 10 bis 20 Gew.-%, in der Zusammensetzung enthalten ist.

26. Verwendung nach einem der Ansprüche 20 bis 25, wobei das Wachs unter tierischen, pflanzlichen, mineralischen und synthetischen Wachsen, wie z.B. Bienenwachs, Carnaubawachs, Candelillawachs, Ouricourywachs, Japanwachs, Korkfaserwachs oder Zuckerrohrwachs, Paraffinwachsen, Lignit, mikrokristallinen Wachsen, Ozokeriten, Polyethylenwachsen, den durch Fischer-Tropsch-Synthese erzeugten Wachsen, Siliconwachsen und deren Gemischen ausgewählt ist.

27. Verwendung nach einem der Ansprüchen 20 bis 26, wobei das Wachs in einem Anteil von 15 bis 60 Gew.-%, bezogen auf das Gesamtgewicht der Zusammensetzung, enthalten ist.

28. Verwendung nach einem der Ansprüche 20 bis 27 in einer Zusammensetzung, die weniger als 20 Gew.-%, vorzugsweise weniger als 5 Gew.-% nichtflüchtiges Kohlenwasserstofflöl enthält.

29. Verwendung nach einem der Ansprüche 20 bis 28 in einer Zusammensetzung, die in Form einer kosmetischen oder dermatologischen Zusammensetzung vorliegt, die einen kosmetisch oder dermatologisch akzeptablen Träger enthält.

30. Verwendung nach einem der Ansprüche 20 bis 29 in einer Zusammensetzung, die in Form einer Schminkzusammensetzung, insbesondere als Make-up, Wangen-Rouge oder Lidschatten, Lippenstift, einer Pflegegrundmasse oder einer fixierenden Grundmasse für die Lippen, eines Pflegeprodukts für die Haut oder einer Sonnenschutzzusammensetzung oder einer selbstbräunenden Zusammensetzung vorliegt.

31. Verfahren zur Begrenzung, Verringerung und/oder Verhinderung des Übergehens einer Schminkzusammensetzung oder Pflegezusammensetzung für die Haut, die Schleimhäute und/oder die Semi-Schleimhäute, insbesondere eines Lippenstifts oder eines Make-ups, wobei die Zusammensetzung in Form einer weichen Paste vorliegt und mindestens 12 Gew.-% Wachs, bezogen auf das Gesamtgewicht der Zusammensetzung, enthält, das darin besteht, in die Zusammensetzung ein flüchtiges Öl und ein Phenylgruppen-haltiges Siliconöl einzubringen.

## Claims

1. Composition which is in the form of a soft paste and includes, in a fatty phase, a volatile oil, a phenylsilicone oil and at least 12 % by weight of wax relative to the total weight of the composition, the composition being obtained by premixing at least a proportion of the constituents, including at least the wax or waxes, heating this premix to a temperature at which it melts and blending the mixture obtained as it cools down to room temperature, the blending being performed at least partially in a mixer-extruder.

2. Composition according to Claim 1, in which the volatile oil is chosen from hydrocarbon oils and cyclic or linear silicone oils, by themselves or mixed.

3. Composition according to either of the preceding claims, in which the volatile oil is chosen from cyclotetradimethylsiloxane, cyclopentadimethylsiloxane, cyclo-hexadimethylsiloxane, methylhexyldimethylsiloxane and isoparaffins, by themselves or mixed.

4. Composition according to one of the preceding claims, in which the volatile oil is present in a proportion of 8-70 % by weight, preferably 30-60 %, relative to the total weight of the composition.

5. Composition according to one of the preceding claims, in which the phenylsilicone oil is chosen from the oils of formula (I) and their mixture: 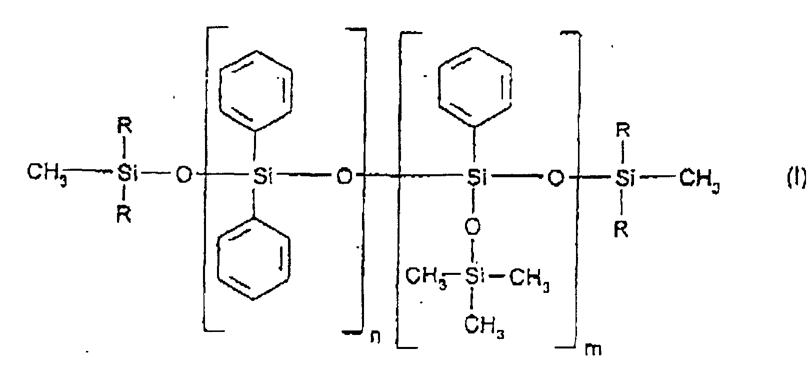 in which
- R is a C₁-C₃₀ alkyl radical, an aryl radical or an aralkyl radical,
- n is an integer between 0 and 100,
- m is an integer between 0 and 100, provided that the sum m+n is between 1 and 100.

6. Composition according to Claim 5, in which the phenylsilicone oil is chosen from the oils of formula (I) for which R is a methyl, ethyl, propyl, isopropyl, decyl, dodecyl, octadecyl, phenyl, tolyl, benzyl or phenethyl radical.

7. Composition according to one of the preceding claims, in which the phenylsilicone oil is present in a proportion of 1-35 % by weight, preferably 10-20 %, in the composition.

8. Composition according to one of the preceding claims, in which the wax has a melting point higher than approximately 45°C, preferably higher than 55°C, and/or a needle penetration number, at 25°C, of between 3 and 40.

9. Composition according to one of the preceding claims, in which the wax is chosen from animal, plant, mineral and synthetic waxes such as beeswax, carnauba, candelilla, ouricoury and Japan wax, cork fibre or sugarcane waxes, paraffin and lignite waxes, microcrystalline waxes, ozokerites, polyethylene waxes, the waxes obtained by Fischer-Tropsch synthesis, silicone waxes, and mixtures thereof.

10. Composition according to one of the preceding claims, in which the wax is present in a proportion of 15-60 % by weight relative to the total weight of the composition.

11. Composition according to one of the preceding claims, exhibiting a dynamic viscosity at 25°C of between 3 and 35 Pa s, measured with a Contraves TV rotary viscometer fitted with an "MS-r4" rotor, at a frequency of 60 Hz.

12. Composition according to one of the preceding claims, including less than 20 % by weight, preferably less than 5 % by weight, of nonvolatile hydrocarbon oil.

13. Composition according to one of the preceding claims, additionally including a particulate phase present in a proportion of 0-35 % by weight, preferably 5-25 % by weight.

14. Composition according to one of the preceding claims, which is in the form of a cosmetic or dermatological composition, including a cosmetically or dermatologically acceptable vehicle.

15. Composition according to one of the preceding claims, which is in the form of a make-up composition, especially a foundation, a blusher or eyeshadow or a lipstick, of a care base or of a fixing base for the lips, of a skincare product or of an antisun or self-tanning composition.

16. Process for the preparation of a composition according to any one of the preceding claims, in which a premix is prepared including at least a proportion of the various constituents of the composition, including at least the wax or waxes, this premix is heated to a temperature at which it melts, if appropriate the remaining constituents are added while the mixture obtained is being blended, and then the mixture obtained is cooled while being blended as it is cooled down to room temperature, the blending being performed at least partially in a mixer-extruder.

17. Process according to Claim 16, in which the volatile oils are added during the cooling stage, preferably at a temperature lower than or equal to approximately 45°C.

18. Process according to either of Claims 16 and 17, in which the heating, blending and cooling operations are carried out in one or more extruders arranged one following the other.

19. Process according to one of Claims 16 to 18, in which the heating, blending and cooling operations are carried out in a single twin-screw extruder.

20. Use of the combination of a volatile oil and of a phenylsilicone oil in a composition in the form of a soft paste, including at least 12 % by weight of wax relative to the total weight of the composition in order to decrease the transfer and/or the migration of the said composition.

21. Use of the combination of a volatile oil and of a phenylsilicone oil in a composition in the form of a soft paste, including a fatty phase containing at least 12 % of wax relative to the total weight of the composition in order to improve the staying power of the said composition.

22. Use according to either of Claims 20 and 21, in which the volatile oil is chosen from hydrocarbon oils and cyclic or linear silicone oils, by themselves or mixed.

23. Use according to one of Claims 20 to 22, in which the volatile oil is present in a proportion of 8-70 % by weight, preferably 30-60 %, in the composition.

24. use according to one of Claims 20 to 23, in which the phenylsilicone oil is chosen from the oils of formula (I) and their mixtures: 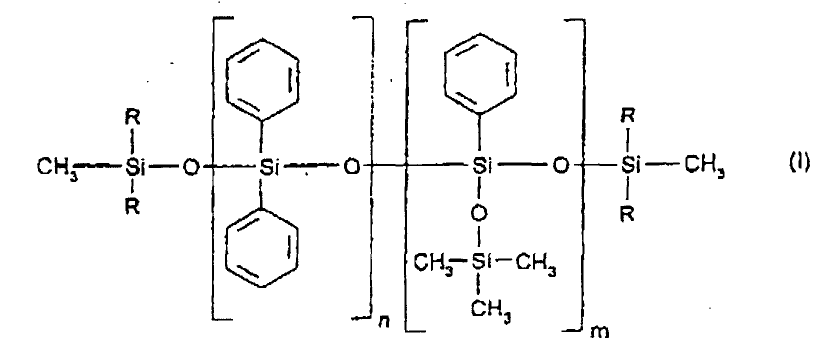 in which
- R is a C₁-C₃₀ alkyl radical, an aryl radical or an aralkyl radical,
- n is an integer between 0 and 100,
- m is an integer between 0 and 100, provided that the sum m+n is between 1 and 100.

25. Use according to one of Claims 20 to 24, in which the phenylsilicone oil is present in a proportion of 1-35 % by weight, preferably 10-20 %, in the composition.

26. Use according to one of Claims 20 to 25, in which the wax is chosen from animal, plant, mineral and synthetic waxes such as beeswax, carnauba, candelilla, ouricoury and Japan wax, cork fibre or sugarcane waxes, paraffin and lignite waxes, microcrystalline waxes, ozokerites, polyethylene waxes, the waxes obtained by Fischer-Tropsch synthesis, silicone waxes, and mixtures thereof.

27. Use according to one of Claims 20 to 26, in which the wax is present in a proportion of 15-60 % by weight relative to the total weight of the composition.

28. Use according to one of Claims 20 to 27, in a composition including less than 20 % by weight, preferably less than 5 % by weight, of nonvolatile hydrocarbon oil.

29. Use according to one of Claims 20 to 28, in a composition which is in the form of a cosmetic or dermatological composition, including a cosmetically or dermatologically acceptable vehicle.

30. Use according to one of Claims 20 to 29 in a composition which is in the form of a make-up composition, especially a foundation, a blusher or eyeshadow or a lipstick, of a care base or of a fixing base for the lips, of a skincare product or of an antisun or self-tanning composition.

31. Process for limiting, reducing and/or preventing the transfer of a make-up or care composition for the skin, mucous membranes and/or semi-mucous membranes, in particular a lipstick or a foundation, the said composition being in the form of a soft paste and comprising at least 12 % by weight of wax relative to the total weight of the composition, this process consisting in introducing a volatile oil and a phenylsilicone oil into the said composition.
